# EUROPEAN PATENT SPECIFICATION

(11) **EP 0 939 071 B1**
(45) Date of publication and mention of the grant of the patent: **30.07.2003**
(21) Application number: 99103578.3
(22) Date of filing: 24.02.1999
(51) Int. Cl.: C07C 19/08, C07C 17/21, C07C 17/00, C07C 17/20, C07C 17/087, C07C 21/18

(54) **Method for producing fluorinated propane**
Verfahren zur Herstellung von fluorierten Propanen
Procédé pour la préparation de propanes fluorées

(30) Priority: 26.02.1998 JP 4508898; 20.04.1998 JP 10958698
(43) Date of publication of application: 01.09.1999
(73) Proprietor: Central Glass Company, Limited, Ube-shi, Yamaguchi-ken 755-0001 (JP)
(72) Inventor: Hibino, Yasuo Chemical Research Center, Kawagoe-shi, Saitama 350-1151 (JP); Tamai, Ryouichi Chemical Research Center, Kawagoe-shi, Saitama 350-1151 (JP); Kaneda, Shouzou Chemical Research Center, Kawagoe-shi, Saitama 350-1151 (JP)
(74) Representative: Manitz, Finsterwald & Partner GbR

(56) References cited:
- EP-A- 0 712 826
- WO-A-97/08117
- WO-A-97/24307
- GB-A- 938 070
- GB-A- 2 313 118
- US-A- 5 710 352
- DATABASE WPI Section Ch, Week 9740 Derwent Publications Ltd., London, GB; Class B05, AN 97-431415 XP002049721 & JP 09 194404 A (CENTRAL GLASS CO LTD) , 29 July 1997
- PATENT ABSTRACTS OF JAPAN vol. 098, no. 005, 30 April 1998 & JP 10 007605 A (CENTRAL GLASS CO LTD), 13 January 1998
- PATENT ABSTRACTS OF JAPAN vol. 098, no. 008, 30 June 1998 & JP 10 067693 A (CENTRAL GLASS CO LTD), 10 March 1998

## Description

The present invention relates to methods for producing 1,1,1,3,3-pentafluoropropane, by fluorinating 1,1,1,3,3-pentachloropropane. 1,1,1,3,3-pentafluoropropane is useful, for example, as a refrigerant and as a foaming agent for producing foamed polyurethane.

There is a method for producing a fluorinated propane by fluorinating a chlorinated compound by hydrogen fluoride in the presence of a catalyst. There is another method for producing a fluorinated propane by adding hydrogen fluoride to a halogenated propene, optionally followed by a fluorination through an exchange of chlorine for fluorine. There is still another method for producing a fluorinated propane by reducing or chlorinating another fluorinated propane by hydrogen or chlorine.

Japanese Patent Unexamined Publication JP-A-6-256235 discloses a method for producing 1,1,1,3,3-pentafluoropropane from CF₃-CClX-CF₂Cl where X is hydrogen or chlorine, by catalytic hydrogenation. A preferable catalyst for this method is a common hydrogenation catalyst. There is disclosed, in published English translation (pp. 1312-1317) of Izvestiya Akademii Nauk SSSR, Otdelenie Khimicheskikh Nauk, No. 8, pp. 1412-1418, August, 1960 (CA 55, 349f), a method for producing 1,1,1,3,3-pentafluoropropane by hydrogenating 1,1,3,3,3-pentafluoro-1-propene in the presence of Pd-Al₂O₃. U.S. Patent No. 2,942,036 discloses a method of hydrogenating 1,2,2-trichloropentafluoropropane to produce 1,1,1,3,3-pentafluoropropane or 1,1,3,3,3-pentafluoro-1-propene or mixtures thereof. U.S. Patent No. 5.574,192 discloses a method for producing 1,1,1,3,3-pentafluoropropane by fluorinating 1,1,1,3,3-pentachloropropane by hydrogen fluoride in a liquid phase in the presence of a catalyst. JP-A-9-002983 discloses a method for producing 1,1,1,3,3-pentafluoropropane by fluorinating 1,1,1,3,3-pentachloropropane by hydrogen fluoride in a gas phase in the presence of a catalyst. JP-A-9-183740 discloses a method for producing 1,1,1,3,3-pentafluoropropane by (a) fluorinating 1,1,1,3,3-pentachloropropane by hydrogen fluoride in a gas phase in the presence of a catalyst, thereby to obtain a reaction mixture of 1-chloro-3,3,3-trifluoropropene and hydrogen chloride produced as a by-product; (b) removing the hydrogen chloride from the reaction mixture; and (c) fluorinating the 1-chloro-3,3,3-trifluoropropene by hydrogen fluoride in a gas phase in the presence of a catalyst into 1,1,1,3,3-pentafluoropropane.

It is known to use (1) an oxyfluoride of aluminum or chromium, which is prepared by fluorinating alumina or chromia (chromium oxide), or (2) a catalyst having at least one metal loaded on a carrier, as a catalyst for fluorinating chlorinated or chlorofluorinated hydrocarbons. Milos Hudlicky, "Chemistry of Organic Fluorine Compounds", 2nd Ed., p. 99 (1976) discloses a reaction of 1,1,2,2-tetrachloroethane with hydrogen fluoride and chlorine at 200°C, using a catalyst having antimony pentachloride adsorbed on an activated carbon, thereby to obtain 1,1,2-trichloro-1,2,2-trifluoroethane (yield: 65%). EP-A1-0712826 discloses that 1,1,1-trifluoroethane is synthesized by fluorinating 1-chloro-1,1-difluoroethane by hydrogen fluoride in the presence of antimony pentachloride supported on an activated carbon. GB-A-938 070 discloses also the use of antimony pentachloride supported on activated carbon in the gas phase to fluorinate a halogenated saturated compound, namely halogenated propane.

GB-A-2 313 118 discloses a two-stage method for the production of 1,1,1,3,3-pentafluoropropane. In the first stage 1,1,1,3,3-pentachloropropane is fluorinated by hydrogen fluoride in the gas phase in the presence of a fluorinating catalyst to obtain essentially 1-chloro-3,3,3-trifluoropropene. In the second stage 1-chloro-3,3,3-trifluoropropene is fluorinated by hydrogen fluoride in the liquid phase in the presence of antimony pentachloride. US-A-5 710 352 discloses a similar two-stage process.

WO 97/08117 discloses a process for preparing fluorinated propane compounds by reacting chlorinated propene compounds with hydrogen fluoride in a liquid phase in the presence of antimony pentachloride.

JP-A-09 194404, JP-A-10 007605 and JP-A-10 067693 disclose the fluorination of halogenated alkanes by hydrogen fluoride in the gas phase in the presence of a fluorinating catalyst to obtain halogenated alkenes.

### SUMMARY OF THE INVENTION

For example, when 1,1,1,3,3-pentachloropropane or 1-chloro-3,3,3-trifluoropropene in the cis or trans form is fluorinated, the aimed product, 1,1,1,3,3-pentafluoropropane (boiling point: 15.3°C), is formed. Upon this, however, 1-chloro-3,3,3-trifluoropropene (its trans-form has a boiling point of 21.0°C) and 1,3,3,3-tetrafluoropropene are often contained in the reaction product of this fluorination, together with the aimed product, as demonstrated in JP-A-9-183740. It is very difficult to separate the aimed product from these compounds by distillation, because the aimed product and these compounds form an azeotropic mixture or the like. Thus, it is very important to decrease the amount of such compounds (the fluorinated propenes) in the reaction product.

It is an object of the present invention to provide a method for producing 1,1,1,3,3-pentafluoropropane, which method is appropriate for the production of the same in an industrial scale.

According to the present invention, there is provided a method for producing 1,1,1,3,3-pentafluoropropane, comprising the steps of:
(a) fluorinating 1,1,1,3,3-pentachloropropane by hydrogen fluoride in a gas phase in the presence of a first fluorination catalyst, thereby obtaining a reaction gas comprising 1-chloro-3,3,3-trifluoropropene; and
(b) fluorinating said reaction gas by hydrogen fluoride in a gas phase by transferring said reaction gas to a reaction zone containing as a second fluorination catalyst an activated carbon supporting thereon antimony pentachloride, thereby obtaining said 1,1,1,3,3-pentafluoropropane.

According to the present invention, it is optional to omit the step (a). In this case, 1-chloro-3,3,3-trifluoropropene is fluorinated by hydrogen fluoride in a gas phase in a reaction zone in which the second fluorination catalyst is present.

As stated above, according to the present invention, the special second fluorination catalyst is used in the step (b). With this, it becomes possible to increase the conversion of the raw material and the selectivity of the aimed fluorinated propane, even if hydrogen chloride is not removed from the reaction gas obtained by the step (a), prior to the step (b). In other words, according to the present invention, it becomes possible to decrease the amount of the above-mentioned raw material and/or intermediate(s) in the reaction product of the step (b). The step (b) may be conducted under atmospheric pressure or pressurized condition.

### DESCRIPTION OF THE PREFERRED EMBODIMENTS

The manner to conduct each of the steps (a) and (b) is not particularly limited, and it may be the fixed bed method or the fluidized bed method.

1-Chloro-3,3,3-trifluoropropene (in the following designated as fluorinated propene) obtained by the step (a) is a compound formed by removing hydrogen chloride from 1,1,1,3,3-pentachloropropane. Furthermore, said fluorinated propene may be in the cis or trans form or a mixture thereof.

The reaction gas obtained by the step (a) contains the fluorinated propene as a main organic component, hydrogen chloride, and hydrogen fluoride unreacted in the step (a). According to the invention, it is possible to conduct the steps (a) and (b) in series, without interruption therebetween. Thus, it is not particularly necessary to identify the organic component of the reaction gas obtained by the step (b).

It is possible to produce the raw material of the invention by conventional methods. The halogenated propane, 1,1,1,3,3-pentachloropropane, may be produced by the following conventional first, second, and third processes. In the first process, vinylidene chloride is reacted with chloroform in the presence of copper-amine catalyst (see M. Kotora et al. (1991) React. Kinet. Catal. Lett., Vol. 44, No. 2, pp. 415-419). In the second process, carbon tetrachloride is reacted with vinyl chloride in the presence of copper-amine catalyst (see M. Kotora et al. (1992) J. of Molecular Catalysis, Vol. 77, pp. 51-60). In the third process, carbon tetrachloride is reacted with vinyl chloride in an isopropanol solvent, in the presence of a ferrous chloride catalyst (see E. N. Zil'berman et al. (1967) J. of Org. Chem. USSR, Vol. 3, pp. 2101-2105).

As stated above, the step (a) is conducted in the presence of a first fluorination catalyst. This catalyst may be selected from fluorinated alumina, fluorinated titania, fluorinated stainless steel and activated carbon, or a catalyst carrying at least one metal on a carrier. The at least one metal is selected from metals of 3, 4, 5, 6, 7, 8, 9 and 13 groups of periodic table, such as aluminum, chromium, manganese, nickel and cobalt. The carrier is selected from alumina, fluorinated alumina, aluminum fluoride and activated carbon. The method for preparing the first fluorination catalyst of this type is not particularly limited. In fact, the carrier may be immersed into a solution of a water-soluble compound (e.g., nitrate and chloride) of the metal, or alternatively the solution may be sprayed on the carrier. After that, the carrier may be dried and then brought into contact with a halogenation agent (e.g., hydrogen fluoride, hydrogen chloride, and chlorofluorohydrocarbon) under a heated condition, thereby to partially or completely halogenate the carrier or the metal loaded thereon. This fluorination for preparing the catalyst is not particularly limited. For example, it is possible to prepare a fluorinated alumina by bringing a hydrogen fluoride gas into contact under a heated condition with an alumina that is commercially available for use as a desiccant or catalyst carrier. Alternatively, it can be prepared by spraying a hydrogen fluoride aqueous solution at about room temperature to an alumina or by immersing an alumina into the solution, followed by drying. The above-mentioned activated carbon as the first fluorination catalyst may be the same as that of the second fluorination catalyst.

As stated above, the second fluorination catalyst of the step (b) is an activated carbon supporting thereon antimony pentachloride.

The method for preparing the second fluorination catalyst is not particularly limited, as long as the antimony pentachloride is attached to an activated carbon as the carrier in the resultant second fluorination catalyst. The second fluorination catalyst can be prepared as follows. It is possible to apply the antimony pentachloride itself, by dropping, spraying, or immersion, to an activated carbon that has been subjected, according to need, to the after-mentioned pretreatment. Alternatively, the antimony pentachloride may be dissolved in an inert solvent. The resultant solution may be applied to an activated carbon by spraying, or an activated carbon may be immersed in the solution. Then, the activated carbon coated with the antimony pentachloride is dried by heating and/or vacuum. After that, the dried activated carbon may be brought into contact, under a heated condition, with hydrogen fluoride, chlorine, hydrogen chloride, or chlorofluorohydrocarbon, to complete the preparation of the second fluorination catalyst. In particular, it is preferable to treat the dried activated carbon with chlorine of at least 1 equivalent at a temperature not lower than 100°C for activating the second fluorination catalyst.

The above-mentioned inert solvent for preparing the second fluorination catalyst is not particularly limited, as long as it can dissolve the antimony pentachloride and does not decompose the same. Examples of this inert solvent are lower alcohols such as methanol, ethanol and isopropanol; ethers such as methyl cellosolve, ethyl cellosolve and diethyl ether; ketones such as acetone and methyl ethyl ketone; aromatic compounds such as benzene, toluene and xylene; esters such as ethyl acetate and butyl acetate; and chlorinated solvents such as methylene chloride, chloroform, tetrachloroethylene and tetrachloroethane; fluorinated solvents such as 1,1-dichloro-1-fluoroethane, 3,3-dichloro-1,1,2,2,3-pentafluoropropane, 1,3-bis(trifluoromethyl) benzene and trifluoromethylbenzene; and the fluorinated (halogenated) propane, which is the starting material, the intermediate and the reaction product in the method of the invention. For example, it is preferable to use fluorinated solvents, such as 3-chloro-1,1,1,3-tetrafluoropropane, 3,3-dichloro-1,1,1-trifluoropropane, 1,3-bis(trifluoromethyl)benzene and trifluoromethylbenzene, as solvents for dissolving antimony pentachloride. Regardless of whether or not the solvent is used in the preparation of the second fluorination catalyst, it is preferable to make the activated carbon carry thereon the antimony pentachloride in the presence of substantially no water, by removing a substance(s) (e.g., water) that is reactive with the antimony pentachloride, from the solvent and the system that makes the activated carbon carry thereon the antimony pentachloride.

The amount of the antimony pentachloride supported on an activated carbon is preferably from 0.1 to 500 parts by weight, more preferably from 1 to 250 parts by weight, per 100 parts by weight of the activated carbon.

The activated carbon used in the present invention is not limited to a particular type. The activated carbon may be prepared from a vegetable raw material such as wood, charcoal, coconut husk coal, palm core coal, or raw ash; a coal such as peat, lignite, brown coal, bituminous coal, or anthracite; a petroleum raw material such as petroleum residue or oil carbon; or a synthetic resin raw material such as carbonated polyvinylidene chloride. The activated carbon may be selected from various commercial activated carbons. Examples of commercial activated carbons that are usable in the invention are an activated carbon having a trade name of BPL GRANULAR ACTIVATED CARBON that is made of bituminous coal and made by TOYO CALGON CO. and coconut husk coals made by Takeda Chemical Industries, Ltd. having trade names of GRANULAR SHIRO SAGI GX, CX and XRC, and a coconut husk coal made by TOYO CALGON CO. having a trade name of PCB. An activated carbon used in the invention is generally in the form of granules. Furthermore, it may be in the form of sphere, fiber, powder or honeycomb. Its shape and size are not particularly limited, and may be decided depending on the reactor.

An activated carbon used in the invention preferably has a large specific surface area. Commercial products of activated carbon will suffice for the invention with respect to specific surface area and micropore volume. In the invention, the specific surface area of the activated carbon is preferably greater than 400 m²/g, more preferably from 800 to 3,000 m²/g. Furthermore, the micropore volume of the activated carbon is preferably greater than 0.1 cm³/g, more preferably from 0.2 to 1.0 cm³/g. When an activated carbon is used as a carrier of the second fluorination catalyst, it is preferable to activate the surface of the carrier and remove ashes therefrom by immersing the activated carbon in a basic aqueous solution of ammonium hydroxide, sodium hydroxide, potassium hydroxide or the like at about room temperature for 10 hr or more or by subjecting the activated carbon to a pretreatment with an acid such as nitric acid, hydrochloric acid or hydrofluoric acid. This pretreatment is conventionally used, upon the use of activated carbon as a catalyst carrier.

In the preparation of the second fluorination catalyst, it is preferable to remove water as much as possible from the carrier by heating, or vacuum, prior to the application of a high valence metal halide to the carrier, in order to prevent deterioration of the halide caused by hydrolysis.

It is possible to prevent the compositional change, the lifetime shortening, the abnormal reaction and the like of the first or second fluorination catalyst during the fluorination (i.e., the above step (a) or (b)) by bringing, prior to the fluorination, the first or second fluorination catalyst into contact with a fluorination agent such as hydrogen fluoride, fluorohydrocarbon or fluorochlorohydrocarbon. In particular, it is preferable to bring the second fluorination catalyst into contact with hydrogen fluoride and/or chlorine, prior to the fluorination, in order to obtain the same advantageous effects. It is preferable to supply chlorine, fluorochlorohydrocarbon or chlorohydrocarbon into the reactor during the fluorination, in order to improve the catalyst lifetime, conversion and yield. In particular, chlorine is preferably supplied in order to improve and maintain the catalyst activity. In fact, it is preferable that 0.1-10 moles of chlorine is present, at least in the reaction zone of the step (b), per 100 moles of the halogenated propane of the step (a).

The fluorination catalyst activity may be inactivated, when, for example, antimony (V) of the catalyst is reduced to antimony (III) by a reducing agent, or when the catalyst surface is covered with a high-boiling-point organic compound(s) by fluorinating a highly polymerizable organic raw material. This inactivation can be prevented by the presence of an oxidizing agent (e.g., chlorine) in the reaction system.

The reaction pressure of the step (a) is preferably from 0 to 2.0 MPa in terms of gauge pressure in order to prevent the liquefaction of the starting organic compound, the intermediate and hydrogen fluoride and to satisfy the physical conditions required in the use of the first fluorination catalyst. It is still more preferably from 0 to 1.0 MPa in terms of gauge pressure.

The step (a) is conducted at a temperature preferably of 180-450°C, more preferably of 200-350°C. If it is lower than 180°C, the reaction rate may become too low. If it is higher than 450°C, it may become difficult to maintain the catalyst activity. The step (b) is conducted at a temperature preferably of 20-300°C, more preferably of 40-180°C. If it is less than 20°C, the reagents may turn into liquid. Furthermore, the reaction rate may become impractically low. The reaction rate becomes high by increasing the reaction temperature. However, if the reaction temperature is increased too much, the catalyst lifetime may become too short. Furthermore, selectivity of the aimed fluorinated propane may become too low by the formation of decomposition products.

The reaction pressure of the step (a) is preferably higher than that of the step (b). With this, the step (a) may be conducted under a pressure higher than normal pressure. Thus, the reaction gas itself is taken out of the first reactor under a pressurized condition. With this, it becomes substantially easier to conduct a cooling for separating the reaction gas obtained by the step (a) into hydrogen chloride and the other components through gas-liquid separation or distillation separation, as compared with a case in which the step (a) is conducted under normal pressure.

In the step (a), it suffices to supply hydrogen fluoride in at least a stoichiometric amount relative to that of the raw material of the step (a), that is, 1,1,1,3,3-pentachloropropane. A molar ratio of hydrogen fluoride to this halogenated propane of at least 3 will suffice to obtain 1-chloro-3,3,3-trifluoropropene by the step (a). Regarding the step (b), the amount of the aimed product, the fluorinated propane, increases by supplying a higher amount of hydrogen fluoride, relative to that of the fluorinated propene as the raw material of the step (b), from the viewpoint of chemical equilibrium. This may, however, cause economic disadvantages such as necessity to enlarge the reactor or the apparatus of the subsequent treatment. Thus, it is preferable in the step (b) to adjust the molar ratio of the fluorinated propene to hydrogen fluoride to a range of 1/50 to 1/1, more preferably of 1/20 to 1/2. In case that the steps (a) and (b) are conducted in series without interruption therebetween, hydrogen fluoride unreacted in the step (a) is introduced into the reaction zone of the step (b). Thus, the supply of hydrogen fluoride in an excessive amount in the step (a) does not cause any particular problem, except the apparatus enlargement. In the step (b), it is optional to further introduce hydrogen fluoride into the reaction zone of the step (b), in addition to the hydrogen fluoride unreacted in the step (a).

Although hydrogen chloride contained in the reaction gas obtained by the step (a) is considered to be an undesirable substance in the step (b) from the viewpoint of chemical equilibrium, it is not particularly necessary in the invention to remove hydrogen chloride from the reaction gas obtained by the step (a), prior to the step (b), as is explained in the following. If the step (b) is conducted after the step (a) in accordance with the invention except in that the second fluorination catalyst of the invention is replaced with one of conventional fluorination catalysts, the chemical composition of the reaction product of the step (b) may vary depending on the ratio of the amount of hydrogen chloride to that of hydrogen fluoride of the reaction gas obtained by the step (a). In contrast with this, the reaction product of the step (b), in which the second fluorination catalyst of the invention is used, does not substantially vary by the presence of hydrogen chloride in the reaction gas obtained by the step (a). In the invention, however, the removal of hydrogen chloride from the reaction gas obtained by the step (a) does not cause any problem, except the complexation of the process.

The reaction gas obtained by the step (a) may contain small amounts of high-boiling-point organic compounds (impurities) formed in the step (a). These organic compounds may make the second fluorination catalyst of the step (b) inferior in catalytic activity. Therefore, it is preferable to remove these organic compounds from the reaction gas obtained by the step (a), in an additional step between the steps (a) and (b). This addition step is not particularly limited. For example, the organic compounds can be removed by (1) adsorption to activated carbon, (2) absorption into sulfuric acid, (3) solvent absorption, or (4) separation by liquefaction through cooling.

The step (b) may be conducted under atmospheric pressure. It is more preferable to conduct the step (b) under a pressurized condition, in order to shift the position of the chemical equilibrium of the fluorination toward the formation of the fluorinated propane. The pressurization in the step (b) may enhance physical adsorption of the reaction substrates on the surface of the second fluorination catalyst. This may increase the reaction rate of the step (b). This advantageous effect of the pressurization may become striking by using an activated carbon having a large surface area as the carrier of the second fluorination catalyst. In fact, the reaction pressure of the step (b) is preferably from 0 to 5.0 MPa in terms of gauge pressure from the viewpoint of pressure resistance of the reactor, the cost. It is more preferably from 0 to 2.0 MPa in terms of gauge pressure, in order to prevent the liquefaction of the starting organic compound, the intermediate and hydrogen fluoride and to satisfy the physical conditions required in the use of the second fluorination catalyst. It is still more preferably from 0 to about 1.0 MPa in terms of gauge pressure. The step (b) may be conducted under substantially about normal pressure.

The contact time of each of the steps (a) and (b) may be from 0.1 to 300 seconds, preferably from 1 to 60 seconds from the viewpoint of the productivity.

The reactor used in each of the steps (a) and (b) is preferably made of a material that is heat resistant and corrosion resistant against hydrogen fluoride, hydrogen chloride and the like, such as iron, stainless steel, Hastelloy, Monel metal or platinum, or a material lined with one of these metals.

In general, it is known that antimony pentachloride and hydrogen fluoride are corrosive against the material of the reactor. In a liquid phase reaction, an antimony halide and hydrogen fluoride become more corrosive by a dynamic solid-liquid contact between these compounds and the material of the reactor. In contrast, the step (b) of the invention is conducted in a gas phase. Thus, it is assumed that, for example, the antimony pentahalide is present in micropores of the carrier, and hydrogen fluoride is present in the form of gas. Therefore, the corrosiveness of antimony pentahalide and hydrogen fluoride is reduced to a great extent in the invention.

The reaction products of the step (b) may be purified by a conventional purification process that is not particularly limited. In this process, for example, the reaction products, together with hydrogen chloride and the unreacted hydrogen fluoride, are taken out of the reactor in the form of gas. Then, they are washed with water and/or a basic solution or subjected to a treatment such as solubility difference separation, extraction separation or distillation, to remove acid gases therefrom. Then, the resultant organic matter may be rectified to obtain the aimed fluorinated propane.

It is optional to connect in series a first reactor for conducting the step (a) with a second reactor for conducting the step (b) in order to conduct the steps (a) and (b) without interruption therebetween. The first reactor may be charged with a fluorinated alumina or activated carbon as the first fluorination catalyst. The first reactor may be adjusted to have a temperature of 200-300°C. The second reactor may be charged with an activated carbon supporting thereon antimony pentachloride, as the second fluorination catalyst. The second reactor may be adjusted to have a temperature of 40-180°C. For example, 1,1,1,3,3-pentachloropropane as the halogenated propane and hydrogen fluoride may be supplied into the first reactor, and a reaction product containing 1,1,1,3,3-pentafluoropropane and hydrogen chloride may be taken out of the second reactor. 1-Chloro-3,3,3-trifluoropropene is mainly produced in the first reactor as the reaction product of the step (a). Then, the reaction gas is allowed to flow into the second reactor. After the step (b), the reaction gas out of the second reactor, which may contain small amounts of 1-chloro-3,3,3-trifluoropropene and 1,3,3,3-tetrafluoropropene, may be purified as follows. At first, hydrogen chloride is separated from the reaction gas of the step (b). Then, hydrogen fluoride of the remainder is absorbed into sulfuric acid or the like. Then, the resultant organic matter is washed and then dried, followed by rectification through distillation or the like, thereby to obtain the aimed fluorinated propane. It should be noted that the purification is not limited to the above.

The aimed product of the step (b), 1,1,1,3,3-pentafluoropropane, has a relative volatility close to 1, relative to key components of 1-chloro-3,3,3-trifluoropropene (trans form) and 1,3,3,3-tetrafluoropropene (cis form). Therefore, generally speaking, it is very difficult to separate these key components from this aimed product by an ordinary distillation. It is, however, possible to separate these key components as an azeotropic low-boiling-point composition from the aimed product by reducing as much as possible the amount of these key components in the reaction gas of the step (b). This separation is made possible in the invention. In fact, as shown in the after-mentioned Examples, it is possible in the invention to obtain a selectivity of at least 90% of 1,1,1,3,3-pentafluoropropane and to reduce the amount of the key components in the reaction gas of the step (b) to not greater than 1%. Therefore, it is possible in the invention to rectify the reaction gas of the step (b) by an ordinary distillation to have a high distillation yield, with no use of a special distillation apparatus. It is possible to separate other components, such as 1-chloro-3,3,3-trifluoropropene (cis form) and 1,3,3,3-tetrafluoropropene (trans form), from 1,1,1,3,3-pentafluoropropane by an ordinary distillation, because they have substantially separate boiling points.

The method of the invention may further comprise the steps of (c) separating the reaction gas obtained by the step (b) into the fluorinated propane and a remainder comprising the fluorinated propene unreacted in the step (b); and (d) transferring the remainder to the reaction zone of the step (b), thereby to fluorinate the fluorinated propene of the remainder by hydrogen fluoride in a gas phase in the reaction zone into the fluorinated propane.

The following nonlimitative catalyst preparations are illustrative of the present invention.

### CATALYST PREPARATION 1

In this catalyst preparation, the first fluorination catalyst to be used in the step (a) was prepared as follows. At first, 300 g of an activated alumina, NKH3-24 (trade name) of SUMITOMO CHEMICAL CO., LTD. having a particle diameter of 2-4 mm and a specific surface area of 340 m²/g, was washed with water to remove powder from its surface. Separately, 115 g of hydrogen fluoride (anhydrous hydrofluoric acid) was dissolved in 1,035 g of water, to prepare a 10% hydrogen fluoride aqueous solution. Then, this solution was gradually poured onto the activated alumina, followed by stirring. After that, it was allowed to stand still for 3 hr, and then the activated alumina was taken out of the solution, then washed with water, then filtered, and then dried for 2 hr in an electric furnace at 200°C. The dried activated alumina in an amount of 150 ml was introduced into a stainless steel (SUS 316L) cylindrical reaction tube having an inside diameter of 2.5 cm and an axial length of 30 cm. The temperature of the reaction tube was increased to 200°C in the furnace, while nitrogen was allowed to flow through the reaction tube. Then, hydrogen fluoride, together with nitrogen, was allowed to flow therethrough, to fluorinate the activated alumina. As this fluorination proceeded, the temperature increased. However, the flow rates of nitrogen and hydrogen fluoride were suitably adjusted to make the temperature not higher than 400°C. After the heat generation terminated, the temperature of the furnace was maintained at 400°C for 2 hr, thereby to prepare a fluorinated alumina as the first fluorination catalyst.

### CATALYST PREPARATION 2

In this catalyst preparation, a first fluorination catalyst to be used in the step (a) was prepared as follows. At first, a Cr(NO₃)₃ solution was prepared by dissolving 30 g of Cr(NO₃)₃·9H₂O into 100 ml of pure water. In this solution 180 ml of a granular activated carbon made by Takeda Chemical Industries, Ltd. having a trade name of GRANULAR SHIRO SAGI GX was immersed for one day and one night. This activated carbon had a particle diameter of 4-6 mm, a surface area of 1,200 m²/g and an average micropore diameter of 18Å. Then, the activated carbon was separated from the solution by filtration, and then was dried for one day and one night at 100°C in a hot-air circulating type oven. The thus obtained chromium-carried activated carbon in an amount of 150 ml was put into a cylindrical reaction tube that was equipped with a heating device and was made of stainless steel (SUS316L) and had a diameter of 2.5 cm and an axial length of 30 cm. The reaction tube temperature was increased to 300°C, while nitrogen gas was allowed to flow therethrough. Then, at the time when it was found that steam flow therefrom stopped, it was started to allow hydrogen fluoride to flow therethrough, together with nitrogen gas. Then, hydrogen fluoride concentration of the mixture of hydrogen fluoride and nitrogen was gradually increased. The reaction tube temperature was further increased to 350 °C. Then, this condition was maintained for 1 hr, thereby to prepare a Cr-carried activated carbon as the first fluorination catalyst.

### CATALYST PREPARATION 3

In this catalyst preparation, a second fluorination catalyst to be used in the step (b) was prepared as follows. At first, a 1-liter glass flask was charged with 0.25 liter of a granular activated carbon of a trade name of TOYO CALGON PCB (4 × 10 meshes) having a surface area of 1,150-1,250 m²/g and a micropore diameter 1.5-2.0 nm (15-20 Å). Then, the glass flask was heated to a temperature of 130-150°C, followed by the removal of water by a vacuum pump. Then, at the time when it was found that steam flow therefrom stopped, it was started to introduce nitrogen gas into the flask to have normal pressure. Then, 125 g of antimony pentachloride was introduced into the flask with a dropping funnel by spending 1 hr with stirring. The obtained activated carbon impregnated with antimony pentachloride was maintained at 150°C for about 1 hr for aging.

### CATALYST PREPARATION 4

In this catalyst preparation, Catalyst Preparation 3 was repeated except in that the introduction of antimony pentachloride and the subsequent aging were omitted, thereby to prepare a first fluorination catalyst (activated carbon) to be used in the step (a).

### CATALYST PREPARATION 5

In this catalyst preparation, a second fluorination catalyst to be used in the step (b) was prepared as follows. At first, a 1-liter round-bottom glass flask was charged with 0.5 liter of a granular activated carbon made by Takeda Chemical Industries, Ltd. having a trade name of GRANULAR SHIRO SAGI G2X (4-6 meshes). This activated carbon had an average surface area of 1,200 m²/g and an average micropore diameter of 1.8 nm (18Å) Then, the flask was heated to a temperature of 130-150°C, followed by the removal of water by a vacuum pump. Then, at the time when it was found that steam flow therefrom stopped, it was started to introduce nitrogen gas into the flask to have normal pressure. Then, 250 g of antimony pentachloride was introduced into the flask with stirring using a dropping funnel. The obtained activated carbon impregnated with antimony pentachloride was maintained at 150°C for about 1 hr for aging.

### CATALYST PREPARATION 6

In this catalyst preparation, a first fluorination catalyst to be used in the step (a) was prepared as follows. At first, a 1-liter glass flask was charged with 0.2 liter of a granular coconut husk coal made by Takeda Chemical Industries, Ltd. having a trade name of GRANULAR SHIRO SAGI GX (4-6 meshes). This granular coal had a surface area of 1,200 m²/g and a micropore diameter of 1.8 nm (18Å). Then, the glass flask was heated to a temperature of 130-150°C, followed by the removal of water by a vacuum pump. Then, at the time when it was found that steam flow therefrom stopped, it was started to introduce nitrogen gas into the flask to have normal pressure, thereby to prepare the first fluorination catalyst.

The following nonlimitative examples are illustrative of the present invention.

### EXAMPLE 1

A method for producing a fluorinated propane was conducted as follows by using a fluorination apparatus in which first and second reactors were connected together in series by piping.

Prior to the introduction of an organic raw material into the fluorination apparatus, the first and second reactors were subjected to respective conditionings. The conditioning of the first reactor was conducted as follows. At first, 150 ml of the first fluorination catalyst (fluorinated alumina) obtained by Catalyst Preparation 1 was put in a cylindrical reaction tube that was equipped with an electric furnace and was made of stainless steel (SUS316L) and had a diameter of 2.5 cm and an axial length of 30 cm. Then, while nitrogen gas was allowed to flow therethrough at a rate of 160 ml/min, the reaction tube temperature was increased to 300°C. Then, while hydrogen fluoride was allowed to flow therethrough at a rate of 0.2 g/min together with nitrogen gas, the reaction tube temperature was further increased to 350°C. Then, this condition was maintained for 1 hr. Then, the reaction tube temperature was decreased to 250°C, and the flow rate of hydrogen fluoride was maintained at 0.2 g/min, thereby to complete the conditioning of the first reactor. The conditioning of the second reactor was conducted as follows. At first, 250 ml of the second fluorination catalyst obtained by Catalyst Preparation 3 was put in a cylindrical reaction tube that was equipped with an electric furnace and was made of stainless steel (SUS316L) and had a diameter of 4.0 cm and an axial length of 30 cm. Then, while nitrogen gas was allowed to flow therethrough at a rate of about 25 ml/min, the reaction tube temperature was increased to 100°C. Then, the supply of nitrogen gas was stopped, and hydrogen fluoride was allowed to flow there through at a rate of 0.2 g/min. This condition was maintained at 100°C for 6 hr. Then, the reaction tube temperature was decreased to 80°C, thereby to complete the conditioning of the second reactor.

Then, the first and second reactors conditioned as above were connected together to prepare a fluorination apparatus. Then, while hydrogen fluoride and chlorine were supplied into the fluorination apparatus at respective rates of 0.2 g/min and 1.3 mg/min, 1,1,1,3,3-pentachloropropane, which was previously vaporized, was supplied into the first reactor at a rate of 0.43 g/min, and at the same time hydrogen fluoride was supplied to the second reactor at a rate of 0.32 g/min, thereby to start the fluorination.

Immediately after the start of the fluorination, the reaction gas at the outlet of the first reactor was sampled. Then, an acid gas containing hydrogen chloride was removed from the sampled gas. With an analysis by gas chromatography of the obtained gas, it was found that the resultant gas contained 97.9% of 1-chloro-3,3,3-trifluoropropene (the molar ratio of the trans form to the cis form was 9/1), 1.2% of 1,3,3,3,-tetrafluoropropene, and 0.9% of 1,1,1,3,3-pentafluoropropane. These percentages and those of the after-mentioned examples are areal percentages in chromatogram.

The reaction was continued under the same conditions. 2 hr from the start of the reaction, the reaction became stable. After that, the reaction gas flowing out of the second reactor was bubbled into water for 4 hr, thereby to remove an acid gas containing hydrogen chloride and hydrogen fluoride, from the reaction gas. Then, the resultant gas component was collected by a dry ice/acetone trap. The obtained organic matter in an amount of 59.8 g was analyzed by gas chromatography. It was found that the resultant gas component contained 0.2% of 1-chloro-3,3,3-trifluoropropene (trans form), 96.2% of 1,1,1,3,3-pentafluoropropane, 0.1% of 1,3,3,3-tetrafluoropropene (trans form), and 1.5% of 1,1,1,3-tetrafluoro-3-chloropropane.

### EXAMPLE 2

In this example, Example 1 was repeated except in that the first fluorination catalyst of the first reactor was replaced with that obtained by Catalyst Preparation 2 and that the temperature of the second reactor was adjusted to 60°C, in place of 100°C.

The reaction gas at the outlet of the first reactor was sampled in the same manner as that of Example 1. Then, an acid gas containing hydrogen chloride was removed from the sampled gas. With an analysis by gas chromatography of the obtained gas, it was found that the resultant gas contained 98.2% of 1-chloro-3,3,3-trifluoropropene (the molar ratio of the trans form to the cis form was 9/1), 1.0% of 1,3,3,3,-tetrafluoropropene, and 0.7% of 1,1,1,3,3-pentafluoropropane.

The reaction was continued under the same conditions. 2 hr from the start of the reaction, the reaction became stable. After that, the reaction gas flowing out of the second reactor was bubbled into water for 4 hr, thereby to remove an acid gas containing hydrogen chloride and hydrogen fluoride, from the reaction gas. Then, the resultant gas component was collected by a dry ice/acetone trap. The obtained organic matter was in an amount of 58.5 g and analyzed by gas chromatography. It was found that the resultant gas component contained 0.4% of 1-chloro-3,3,3-trifluoropropene (trans form), 91.6% of 1,1,1,3,3-pentafluoropropane, 0.1% of 1,3,3,3-tetrafluoropropene (trans form), and 5.1% of 1,1,1,3-tetrafluoro-3-chloropropane.

### EXAMPLE 3

The conditioning of the first reactor was conducted by putting the first fluorination catalyst obtained by Catalyst Preparation 4 in a cylindrical reaction tube that was the same as that of Example 1 and then by increasing the reaction tube temperature to 250°C, while nitrogen gas was allowed to flow through the reaction tube at a rate of 20 ml/min. The conditioning of the second reactor was modified as follows. At first, 150 ml of the second fluorination catalyst obtained by Catalyst Preparation 5 was put in a cylindrical reaction tube that was the same as that of the first reactor of Example 1. Then, while nitrogen gas was allowed to flow therethrough at a rate of 20 ml/min, the reaction tube temperature was increased to 100°C. Then, hydrogen fluoride was allowed to flow therethrough at a rate of 0.25 g/min for 1 hr, and chlorine was allowed to flow therethrough at a rate of 0.3 g/min for 1 hr. After that, the reaction tube temperature was lowered to 80°C, thereby to complete the conditioning of the second reactor.

Then, the first and second reactors conditioned as above were connected together to prepare a fluorination apparatus. The fluorination was conducted by introducing hydrogen fluoride and 1,1,1,3,3-pentachloropropane into the first reactor. The molar ratio of hydrogen fluoride to this propane was 20/1. The reaction gas formed in the first reactor was continuously introduced into the second reactor. 2 hr after the start of the fluorination, the reaction became stable. After that, the reaction was further continued for 4 hr. Then, the reaction gas component taken out of the second reactor was collected and then analyzed in the same manners as those of Example 1. The obtained organic matter was in an amount of 29.2 g. It was found that the reaction gas component from the second reactor contained 0.1% of 1-chloro-3,3,3-trifluoropropene (trans form), 95.6% of 1,1,1,3,3-pentafluoropropane, 0.5% of 1,3,3,3-tetrafluoropropene (trans form), 0.6% of 1,1,1,3-tetrafluoro-3-chloropropane, 0.1% of 1,1,1-trifluoro-3,3-dichloropropane, and 3.1% of others. It was found that the reaction gas component (intermediates) from the first reactor contained 94.5% of 1-chloro-3,3,3-trifluoropropene (the molar ratio of the trans form to the cis form was 10/1), 0.4% of 1,1,1,3,3-pentafluoropropane, 1.4% of 1,3,3,3-tetrafluoropropene (trans/cis), 0.1% of 1,1,1,3-tetrafluoro-3-chloropropane, 0.1% of 1,1,1-trifluoro-3,3-dichloropropane, and 3.5% of others.

### EXAMPLE 4

In this example, the fluorination was conducted by using only a second reactor of the invention. At first, 0.25 liter of the second fluorination catalyst obtained by Catalyst Preparation 3 was put in a cylindrical reaction tube that was the same as that of Example 1. Then, while nitrogen gas was allowed to flow therethrough at a rate of 25 ml/min, the reaction tube temperature was increased to 100°C. Then, the supply of nitrogen gas was stopped, and at the same time hydrogen fluoride was allowed to flow therethrough at a rate of about 0.22 g/min. This condition was maintained at 100°C for 6 hr. Then, the reaction tube temperature was decreased to 80°C. Then, hydrogen chloride, chlorine and hydrogen fluoride were supplied into the second reactor at respective rates of 0.15 g/min, 1.3 mg/min and 0.30 g/min. Furthermore, 1-chloro-3,3,3-trifluoropropene (the molar ratio of the trans form to the cis form was 89/11), which was previously vaporized, was supplied into the second reactor at a rate of 0.13 g/min, thereby to start the fluorination. In fact, the molar ratio of hydrogen fluoride to this propene was 15/1.

1 hr from the start of the fluorination, the reaction became stable. After that, the reaction gas flowing out of the second reactor was bubbled into water for 4 hr, thereby to remove an acid gas containing hydrogen chloride and hydrogen fluoride, from the reaction gas. Then, the resultant gas component was collected by a dry ice/acetone trap. The obtained organic matter was in an amount of 30.9 g and analyzed by gas chromatography. With this, it was found that the resultant gas component contained 0.2% of 1-chloro-3,3,3-trifluoropropene (trans form), 96.5% of 1,1,1,3,3-pentafluoropropane, 0.1% of 1,3,3,3-tetrafluoropropene (trans form), and 1.2% of 1,1,1,3-tetrafluoro-3-chloropropane. The reaction was further continued, and it was found that the catalytic activity of the second fluorination catalyst was maintained for at least 350 hr.

### EXAMPLE 5

In this example, the fluorination was conducted by using only a second reactor of the invention. The second reactor was conditioned as follows. At first, 0.25 liter of the second fluorination catalyst obtained by Catalyst Preparation 3 was put in a cylindrical reaction tube. This reaction tube made of a stainless steel (SUS316L) had a diameter of 4.0 cm and an axial length of 30 cm and was equipped with a pressure regulating valve and a heating device. Then, while nitrogen gas was allowed to flow therethrough at a rate of about 25 ml/min, the reaction tube temperature was increased to 100°C. Then, the supply of nitrogen gas was stopped, and at the same time hydrogen fluoride was allowed to flow therethrough at a rate of 0.22 g/min. This condition was maintained at 100°C for 6 hr, thereby to complete the conditioning of the second reactor. Then, the reaction tube temperature was increased to 180°C. Then, hydrogen fluoride was supplied into the second reactor at a rate of 0.20 g/min. Furthermore, 1-chloro-3,3,3-trifluoropropene (the molar ratio of the trans form to the cis form was 89/11), which was previously vaporized, was supplied into the second reactor at a rate of 0.13 g/min, thereby to start the fluorination. In fact, the molar ratio of hydrogen fluoride to this propene was 10/1.

1 hr from the start of the fluorination, the reaction became stable. After that, the reaction gas flowing out of the second reactor was bubbled into water, thereby to remove an acid gas from the reaction gas. Then, the resultant gas component was collected by a dry ice/acetone trap. The obtained organic matter was analyzed by gas chromatography. The results of this analysis are shown in Table.

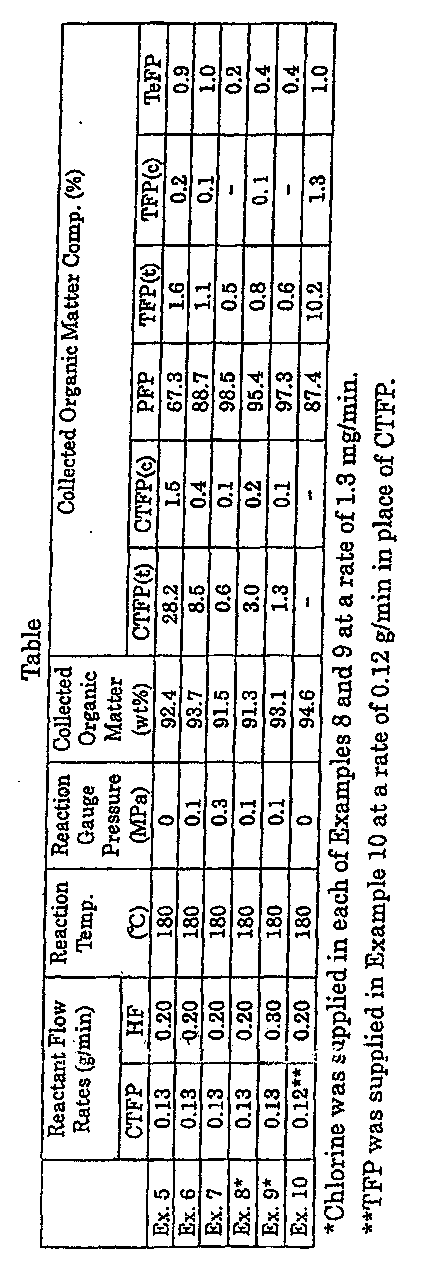

In Table, CTFP, PFP, TFP and TeFP respectively represent 1-chloro-3,3,3-trifluoropropene, 1,1,1,3,3-pentafluoropropane, 1,3,3,3-tetrafluoropropene and 1,1,1,3-tetrafluoro-3-chloropropane, and t and c in parentheses represent trans and cis forms, respectively.

### EXAMPLE 6

In this example, additional procedures were conducted after Example 5, as follows. After the collection of the reaction gas in Example 5, the reaction of Example 5 was terminated. Then, the temperature of the reaction tube (second reactor) was lowered to room temperature. After that, the conditioning of the second reactor was conducted again in the same manner as in Example 5. Then, the fluorination of Example 5 was repeated except in that the reaction pressure (gauge pressure) was adjusted to 0.1 MPa with the pressure regulating valve, as shown in Table. Then, the reaction gas was collected and then analyzed in the same manners as those of Example 5. The results are shown in Table.

### EXAMPLE 7

In this example, additional procedures were conducted after Example 6, as follows. After the collection of the reaction gas in Example 6, the reaction of Example 6 was terminated. Then, the temperature of the reaction tube (second reactor) was lowered to room temperature. After that, the conditioning of the second reactor was conducted again in the same manner as in Example 5. Then, the fluorination of Example 5 was repeated except in that the reaction pressure (gauge pressure) was adjusted to 0.3 MPa with the pressure regulating valve, as shown in Table. Then, the reaction gas was collected and then analyzed in the same manners as those of Example 5. The results are shown in Table.

### EXAMPLE 8

In this example, additional procedures were conducted after Example 7, as follows. After the collection of the reaction gas in Example 7, the reaction of Example 7 was terminated. Then, the temperature of the reaction tube (second reactor) was lowered to room temperature. After that, the conditioning of the second reactor was conducted again in the same manner as in Example 5. Then, the fluorination of Example 5 was repeated except in that chlorine was allowed to flow through the reaction tube at a rate of 1.3 mg/min together with CTFP and HF and that the reaction pressure (gauge pressure) was adjusted to 0.1 MPa with the pressure regulating valve, as shown in Table. Then, the reaction gas was collected and then analyzed in the same manners as those of Example 5. The results are shown in Table.

### EXAMPLE 9

In this example, additional procedures were conducted after Example 8, as follows. After the collection of the reaction gas in Example 8, the reaction of Example 8 was terminated. Then, the temperature of the reaction tube (second reactor) was lowered to room temperature. After that, the conditioning of the second reactor was conducted again in the same manner as in Example 5. Then, the fluorination of Example 5 was slightly modified as shown in Table. In fact, the molar ratio of hydrogen fluoride to CTFP was 15/1. Then, the reaction gas was collected and then analyzed in the same manners as those of Example 5. The results are shown in Table.

### EXAMPLE 10

In this example, additional procedures were conducted after Example 9, as follows. After the collection of the reaction gas in Example 9, the reaction of Example 9 was terminated. Then, the temperature of the reaction tube (second reactor) was lowered to room temperature. After that, the conditioning of the second reactor was conducted again in the same manner as in Example 5. Then, the fluorination of Example 5 was repeated except in that 1,3,3,3-tetrafluoropropene (the molar ratio of the trans form to the cis form was 80/20) was supplied to the reaction tube at a rate of 0.12 g/min in place of CTFP. Then, the reaction gas was collected and then analyzed in the same manners as those of Example 5. The results are shown in Table. Throughout the reactions of Examples 5-10, the starting organic matter was fluorinated for at least 200 hr in total. The catalytic activity of the second fluorination catalyst was maintained throughout this fluorination.

### EXAMPLE 11

At first, 150 ml of the first fluorination catalyst obtained by Catalyst Preparation 6 was put in a cylindrical reaction tube (first reactor) equipped with an electric furnace. This reaction tube made of a stainless steel (SUS316L) had a diameter of 2.5 cm and an axial length of 30 cm. Then, while nitrogen gas was allowed to flow therethrough at a rate of 160 ml/min, the reaction tube temperature was increased to 200°C. Then, hydrogen fluoride was allowed to flow therethrough at a rate of about 0.2 g/min together with nitrogen gas. Under this condition, the reaction tube temperature was increased to 250°C. Then, the flow rate of hydrogen fluoride was adjusted to 0.75 g/min, and 1,1,1,3,3-pentachloropropane was supplied to the reaction tube at a rate of 0.42 g/min. The pressure of the reaction system was adjusted to 0.8 MPa by using a pressure regulating valve of the reaction tube.

2 hr from the start of the fluorination the reaction became stable. After that, the reaction gas flowing out of the first reactor was bubbled into water, thereby to remove an acid gas from the reaction gas. Then, the resultant gas component was collected by a dry ice/acetone trap. The obtained organic matter was analyzed by gas chromatography. With this, it was found that the resultant gas component contained 84.6% of 1-chloro-3,3,3-trifluoropropene (trans form), 11.7% of 1-chloro-3,3,3-trifluoropropene (cis form), 1.0% of 1,3,3,3-tetrafluoropropene (trans form), and 0.3% of 1,1,1,3,3-pentafluoropropane.

Separately, 150 ml of the second fluorination catalyst obtained by Catalyst Preparation 5 was put in a cylindrical reaction tube (second reactor) that was the same as the above first reactor of this example. Then, while nitrogen gas was allowed to flow therethrough at a rate of about 160 ml/min, the reaction tube temperature was increased to 80°C. Then, chlorine gas was allowed to flow therethrough at a rate of 0.6 g/min for 1 hr, and furthermore hydrogen fluoride was introduced thereinto at a rate of 0.2 g/min for 1 hr. Under this condition, the reaction tube temperature was increased to 100°C and then decreased to 80°C. After the reaction of the first reactor became stable, the reaction gas from the first reactor was introduced into the second reactor under normal pressure.

2 hr from the start of the fluorination in the second reactor, the reaction became stable. After that, the reaction gas flowing out of the first reactor was bubbled into water, thereby to remove an acid gas from the reaction gas. Then, the resultant gas component was collected by a dry ice/acetone trap. The obtained organic matter was analyzed by gas chromatography. With this, it was found that the resultant gas component contained 0.1% of 1-chloro-3,3,3-trifluoropropene (trans form), 97.4% of 1,1,1,3,3-pentafluoropropane, 0.2% of 1,3,3,3-tetrafluoropropene (trans form), and 0.3% of 1,1,1,3-tetrafluoro-3-chloropropane.

Advantages of the invention are further discussed as follows. The second fluorination catalyst of the invention, which comprises an activated carbon supporting thereon a halide of a high valence metal, has the following characteristics. It has catalytic activity at low temperature and in a gas phase reaction under low pressure and further has long lifetime. It is possible to obtain a saturated compound (fluorinated propane), particularly 1,1,1,3,3-pentafluoropropane by the catalytic activity of the second fluorination catalyst. The selectivity of the aimed reaction product (e.g., 1,1,1,3,3-pentafluoropropane) becomes high, and the fluorination of the step (b) proceeds even if the reaction system contains hydrogen chloride, by the use of the second fluorination catalyst. Thus, it becomes possible to simplify the process for purifying the fluorinated propane and to prevent the increase of the energy cost and the complexation of the process caused by the recycling of the unreacted raw material(s). It is easily possible to reactivate or, according to need, dump the inactivated first and second catalysts, because the separation of these catalysts from the organic matter(s) is easy in a gas phase reaction. Corrosion of the reactor used in the invention (gas phase reaction) is substantially less than that used in a liquid phase reaction. According to the invention, it becomes possible to conduct the step (b) by using the reaction gas itself obtained by the step (a), without the treatment of this reaction gas. Thus, it becomes possible to omit or simplify the intermediate purification process. The reaction device used in the invention is simple in structure and can easily be designed, because each of the steps (a) and (b) is a gas phase reaction. In the invention, it is easy to control the reaction and optimize the process, because the fluorination of the invention is of two steps. Thus, the invention is appropriate for the production of a fluorinated propane in an industrial scale.

## Claims

1. A method for producing 1,1,1,3,3-pentafluoropropane, comprising the steps of:
(a) fluorinating 1,1,1,3,3-pentachloropropane by hydrogen fluoride in a gas phase in the presence of a first fluorination catalyst, thereby obtaining a reaction gas comprising 1-chloro-3,3,3-trifluoropropene; and
(b) fluorinating said reaction gas by hydrogen fluoride in a gas phase by transferring said reaction gas to a reaction zone containing as a second fluorination catalyst an activated carbon supporting thereon antimony pentachloride, thereby obtaining said 1,1,1,3,3-pentafluoropropane.

2. A method according to claim 1, wherein said first fluorination catalyst of the step (a) comprises a carrier and at least one metal loaded thereon.

3. A method according to claim 2, wherein said carrier is selected from the group consisting of alumina, fluorinated alumina, aluminum fluoride, and activated carbon, and wherein said at least one metal is selected from the group consisting of metals of 3, 4, 5, 6, 7, 8, 9, and 13 groups of periodic table.

4. A method according to claim 1, wherein said first fluorination catalyst of the step (a) is selected from the group consisting of fluorinated alumina, fluorinated titania, fluorinated stainless steel, and activated carbon.

5. A method according to claim 1, wherein said reaction gas obtained by the step (a) is free of a compositional change until the step (b).

6. A method according to claim 1, wherein said reaction gas obtained by the step (a) further comprises hydrogen chloride gas.

7. A method according to claim 1, wherein, prior to the step (b), an organic impurity is removed from said reaction gas.

8. A method according to claim 1, wherein, prior to the step (b), a composition is prepared by adding hydrogen fluoride and/or chlorine to said reaction gas obtained by the step (a), and then the step (b) is conducted by transferring said composition to said reaction zone.

9. A method according to claim 1, wherein the steps (a) and (b) are respectively conducted at a first temperature of 180-450°C and a second temperature of 20-300°C, said first temperature being higher than said second temperature, and wherein a reaction pressure of each of the steps (a) and (b) is in a range of 0-1.0 MPa in terms of gauge pressure.

10. A method according to claim 1, wherein 0.1-10 moles of chlorine is present in said reaction zone of the step (b), per 100 moles of said at least one compound of the step (a).

11. A method according to claim 1, wherein said method further comprises the steps of:
(c) separating a reaction mixture obtained by the step (b) into said fluorinated propane and a remainder comprising said 1-chloro-3,3,3-trifluoropropene unreacted in the step (b); and
(d) transferring said remainder to said reaction zone of the step (b), thereby to fluorinate said 1-chloro-3,3,3-trifluoropropene of said remainder by hydrogen fluoride in a gas phase in said reaction zone into said fluorinated propane.

12. A method according to claim 1, wherein said antimony pentachloride of said second fluorination catalyst is in an amount of 0.1-500 parts by weight per 100 parts by weight of said activated carbon.

13. A method for producing 1,1,1,3,3-pentafluoropropane according to claim 1 said method comprising the steps of:
(a) connecting together first and second reactors in series, said first reactor being charged with a fluorinated alumina or activated carbon and having a temperature of 200-300°C, said second reactor having a temperature of 40-180°C and being charged with an activated carbon supporting thereon antimony pentachloride;
(b) supplying 1,1,1,3,3-pentachloropropane and hydrogen fluoride into said first reactor; and
(c) taking a reaction product formed in said second reactor, out of said second reactor, said reaction product comprising 1,1,1,3.3-pentafluoropropane and hydrogen chloride.

14. A method for producing 1,1,1,3,3-pentafluoropropane, comprising the step of fluorinating 1-chloro-3,3,3-trifluoropropene by hydrogen fluoride in a gas phase in a reaction zone containing as a fluorination catalyst an activated carbon supporting thereon antimony pentachloride, thereby obtaining said 1,1,1,3,3-pentafluoropropane.

15. A method according to claim 14, wherein said step is conducted at a temperature of 20-300°C under a pressure of 0-1.0 MPa in terms of gauge pressure.

16. A method according to claim 14, wherein 0.1-10 moles of chlorine is present in said reaction zone, per 100 moles of said 1-chloro-3,3,3-trifluoropropene.

## Patentansprüche

1. Verfahren zur Herstellung von 1,1,1,3,3-Pentafluorpropan, das die Schritte aufweist:
(a) Fluorieren von 1,1,1,3,3-Pentachlorpropan mit Fluorwasserstoff in einer Gasphase in Anwesenheit eines ersten Fluorierungskatalysators, wodurch ein Reaktionsgas erhalten wird, das 1-Chlor-3,3,3-trifluorpropen enthält; und
(b) Fluorieren des Reaktionsgases mit Fluorwasserstoff in einer Gasphase, indem das Reaktionsgas in eine Reaktionszone überführt wird, die als einen zweiten Fluorierungskatalysator eine Aktivkohle mit darauf aufgebrachtem Antimonpentachlorid enthält, wodurch das 1,1,1,3,3-Pentafluorpropan erhalten wird.

2. Verfahren nach Anspruch 1, bei dem der erste Fluorierungskatalysator der Stufe (a) einen Träger und wenigstens ein darauf aufgebrachtes Metall enthält.

3. Verfahren nach Anspruch 2, bei dem der Träger aus der Gruppe Aluminiumoxid, fluoriertes Aluminiumoxid, Aluminiumfluorid und Aktivkohle ausgewählt ist und das wenigstens eine Metall aus der Gruppe der Metalle der 3., 4., 5., 6., 7., 8., 9. und 13. Gruppe des Periodensystems ausgewählt ist.

4. Verfahren nach Anspruch 1, bei dem der erste Fluorierungskatalysator der Stufe (a) aus der Gruppe fluoriertes Aluminiumoxid, fluoriertes Titandioxid, fluorierter rostfreier Stahl und Aktivkohle ausgewählt ist.

5. Verfahren nach Anspruch 1, bei dem das in Stufe (a) erhaltene Reaktionsgas bis zur Stufe (b) nicht in der Zusammensetzung geändert wird.

6. Verfahren nach Anspruch 1, bei dem das in Stufe (a) erhaltene Reaktionsgas außerdem Chlorwasserstoffgas enthält.

7. Verfahren nach Anspruch 1, bei dem vor der Stufe (b) eine organische Verunreinigung aus dem Reaktionsgas entfernt wird.

8. Verfahren nach Anspruch 1, bei dem vor der Stufe (b) eine Zusammensetzung hergestellt wird durch Zugabe von Fluorwasserstoff und/oder Chlor zu dem in Stufe (a) erhaltenen Reaktionsgas und dann die Stufe (b) durchgeführt wird, indem die Zusammensetzung in die Reaktionszone überführt wird.

9. Verfahren nach Anspruch 1, bei dem die Stufe (a) und (b) bei einer ersten Temperatur von 180-450°C bzw. einer zweiten Temperatur von 20-300°C durchgeführt wird, wobei die erste Temperatur höher ist als die zweite Temperatur, und bei dem ein Reaktionsdruck jeder der Stufen (a) und (b) in einem Bereich von 0-1,0 MPa als Manometerdruck liegt.

10. Verfahren nach Anspruch 1, bei dem 0,1-10 mol Chlor je 100 mol der wenigstens einen Verbindung der Stufe (a) in der Reaktionszone der Stufe (b) anwesend sind.

11. Verfahren nach Anspruch 1, das außerdem die Schritte aufweist:
(c) Trennen einer in Stufe (b) erhaltenen Reaktionsmischung in das fluorierte Propan und einen Rest, der in Stufe (b) nicht umgesetztes 1-Chlor-3,3,3-trifluorpropen enthält; und
(d) Überführen des Restes zu der Reaktionszone von Stufe (b), um dadurch das 1-Chlor-3,3,3-trifluorpropen des Restes mit Fluorwasserstoff in einer Gasphase in der Reaktionszone zu fluoriertem Propan zu fluorieren.

12. Verfahren nach Anspruch 1, bei dem das Antimonpentachlorid des zweiten Fluorierungskatalysators in einer Menge von 0,1-500 Gewichtsteilen je 100 Gewichtsteile Aktivkohle vorliegt.

13. Verfahren zur Herstellung von 1,1,1,3,3-Pentafluorpropan nach Anspruch 1, das die Schritte aufweist:
(a) Ein erster und zweiter Reaktor werden in Reihe miteinander verbunden, wobei der erste Reaktor mit einem fluorierten Aluminiumoxid oder Aktivkohle beladen ist und eine Temperatur von 200-300°C hat, der zweite Reaktor eine Temperatur von 40-180°C hat und mit einer Aktivkohle mit darauf aufgebrachtem Antimonpentachlorid beladen ist;
(b) 1,1,1,3,3-Pentachlorpropan und Fluorwasserstoff werden dem ersten Reaktor zugeführt; und
(c) ein in dem zweiten Reaktor gebildetes Reaktionsprodukt wird aus dem zweiten Reaktor genommen, wobei das Reaktionsprodukt 1,1,1,3,3-Pentafluorpropan und Chlorwasserstoff enthält.

14. Verfahren zur Herstellung von 1,1,1,3,3-Pentafluorpropan, das die Stufe der Fluorierung von 1-Chlor-3,3,3-trifluorpropen mit Fluorwasserstoff in einer Gasphase in einer Reaktionszone aufweist, die als einen Fluorierungskatalysator eine Aktivkohle mit darauf aufgebrachtem Antimonpentachlorid enthält, wodurch 1,1,1,3,3-Pentafluorpropan erhalten wird.

15. Verfahren nach Anspruch 14, bei dem die Stufe bei einer Temperatur von 20-300°C unter einem Druck von 0-1,0 MPa als Manometerdruck durchgeführt wird.

16. Verfahren nach Anspruch 14, bei dem 0,1-10 mol Chlor in der Reaktionszone je 100 mol 1-Chlor-3,3,3-trifluorpropen anwesend sind.

## Revendications

1. Procédé pour la production de 1,1,1,3,3-pentafluoropropane, comprenant les étapes consistant :
(a) à fluorer du 1,1,1,3,3-pentachloropropane par du fluorure d'hydrogène en phase gazeuse en présence d'un premier catalyseur de fluoration, ce qui permet d'obtenir un gaz réactionnel comprenant du 1-chloro-3,3,3-trifluoropropène ; et
(b) à fluorer ledit gaz réactionnel par du fluorure d'hydrogène en phase gazeuse en transférant ledit gaz réactionnel à une zone réactionnelle contenant, comme seconde catalyseur de fluoration, un charbon actif portant du pentachlorure d'antimoine, ce qui permet d'obtenir ledit 1,1,1,3,3-pentafluoropropane.

2. Procédé suivant la revendication 1, dans lequel ledit premier catalyseur de fluoration de l'étape (a) comprend un support sur lequel est chargé au moins un métal.

3. Procédé suivant la revendication 2, dans lequel ledit support est choisi dans le groupe consistant en l'alumine, une alumine fluorée, le fluorure d'aluminium et le charbon actif, et dans lequel ledit au moins un métal est choisi dans le groupe consistant en les métaux des Groupes 3, 4, 5, 6, 7, 8, 9 et 13 du Tableau Périodique.

4. Procédé suivant la revendication 1, dans lequel ledit premier catalyseur de fluoration de l'étape (a) est choisi dans le groupe consistant en une alumine fluorée, un oxyde de titane fluoré, un acier inoxydable fluoré et le charbon actif.

5. Procédé suivant la revendication 1, dans lequel ledit gaz réactionnel obtenu par l'étape (a) est dépourvu de variations de composition jusqu'à l'étape (b).

6. Procédé suivant la revendication 1, dans lequel ledit gaz réactionnel obtenu par l'étape (a) comprend en outre du chlorure d'hydrogène gazeux.

7. Procédé suivant la revendication 1, dans lequel, avant l'étape (b), une impureté organique est éliminée dudit gaz réactionnel.

8. Procédé suivant la revendication 1, dans lequel, avant l'étape (b), une composition est préparée en ajoutant du fluorure d'hydrogène et/ou du chlore audit gaz réactionnel obtenu par l'étape (a), puis l'étape (b) est mise en oeuvre en transférant ladite composition à ladite zone réactionnelle.

9. Procédé suivant la revendication 1, dans lequel les étapes (a) et (b) sont mises en oeuvre respectivement à une première température de 180 à 450°C et une seconde température de 20 à 300°C, ladite première température étant supérieure à ladite seconde température, et dans lequel la pression réactionnelle de chacune des étapes (a) et (b) est comprise dans l'intervalle de 0 à 1,0 mPa en termes de pression manométrique.

10. Procédé suivant la revendication 1, dans lequel une quantité de 0,1 à 10 moles de chlore est présente dans ladite zone réactionnelle de l'étape (b), pour 100 moles dudit au moins un composé de l'étape (a).

11. Procédé suivant la revendication 1, dans lequel ledit procédé comprend en outre les étapes consistant :
(c) à séparer le mélange réactionnel obtenu par l'étape (b) en ledit propane fluoré et un résidu comprenant ledit 1-chloro-3,3,3-trifluoropropène n'ayant pas réagi dans l'étape (b) ; et
(d) à transférer ledit résidu à ladite zone réactionnelle de l'étape (b), pour fluorer ainsi ledit 1-chloro-3,3,3-trifluoropropène dudit résidu par le fluorure d'hydrogène en phase gazeuse dans ladite zone réactionnelle en ledit propane fluoré.

12. Procédé suivant la revendication 1, dans lequel ledit pentachlorure d'antimoine dudit second catalyseur de fluoration est présent en une quantité de 0,1 à 500 parties en poids pour 100 parties en poids dudit charbon actif.

13. Procédé pour la production de 1,1,1,3,3-pentafluoropropane suivant la revendication 1, ledit procédé comprenant les étapes consistant :
(a) à connecter ensemble des premier et second réacteurs en série, ledit premier réacteur recevant une alumine fluorée ou du charbon actif et étant à une température de 200 à 300°C, ledit second réacteur étant à une température de 40 à 180°C et recevant un charbon actif portant du pentachlorure d'antimoine ;
(b) à introduire du 1,1,1,3,3-pentachloropropane et du fluorure d'hydrogène dans ledit premier réacteur ; et
(c) à évacuer un produit de réaction formé dans ledit second réacteur, hors dudit second réacteur, ledit produit de réaction comprenant du 1,1,1,3,3-pentafluoropropane et du chlorure d'hydrogène.

14. Procédé pour la production de 1,1,1,3,3-pentafluoropropane, comprenant l'étape consistant à fluorer du 1-chloro-3,3,3-trifluoropropène par du fluorure d'hydrogène en phase gazeuse dans une zone réactionnelle contenant, comme catalyseur de fluoration, un charbon actif portant du pentachlorure d'antimoine, ce qui permet d'obtenir ledit 1,1,1,3,3-pentafluoroprane.

15. Procédé suivant la revendication 14, dans lequel ladite étape est mise en oeuvre à une température comprise dans l'intervalle de 20 à 300°C sous une pression de 0 à 1,0 mPa en termes de pression manométrique.

16. Procédé suivant la revendication 14, dans lequel une quantité de 0,1 à 10 moles de chlore est présente dans ladite zone réactionnelle, pour 100 moles dudit 1-chloro-3,3,3-trifluoropropène.
